(19) Europäisches Patentamt
European Patent Office
Office européen des brevets

(11) Publication number : **0 488 030 B1**

(12) # EUROPEAN PATENT SPECIFICATION

(45) Date of publication of patent specification : **22.09.93 Bulletin 93/38**

(51) Int. Cl.$^5$ : **G03C 1/34,** C07D 277/62, C07D 293/12

(21) Application number : **91119737.4**

(22) Date of filing : **19.11.91**

(54) **Photographic materials with enhanced latent image stability.**

(30) Priority : **30.11.90 US 620982**

(43) Date of publication of application : **03.06.92 Bulletin 92/23**

(45) Publication of the grant of the patent : **22.09.93 Bulletin 93/38**

(84) Designated Contracting States : **BE CH DE FR GB IT LI NL**

(56) References cited :
**EP-A- 0 410 753**
**US-A- 3 954 478**

(73) Proprietor : **EASTMAN KODAK COMPANY**
**343 State Street**
**Rochester, New York 14650-2201 (US)**

(72) Inventor : **Bonheyo, George Lee, c/o EASTMAN KODAK COMPANY**
**Patent Department, 343 State Street**
**Rochester, New York 14650-2201 (US)**
Inventor : **Leone, Ronald Edmund, c/o EASTMAN KODAK COMPANY**
**Patent Department, 343 State Street**
**Rochester, New York 14650-2201 (US)**
Inventor : **Lok, Roger, c/o EASTMAN KODAK COMPANY**
**Patent Department, 343 State Street**
**Rochester, New York 14650-2201 (US)**

(74) Representative : **Brandes, Jürgen, Dr. rer. nat. et al**
**Wuesthoff & Wuesthoff, Patent- und Rechtsanwälte, Schweigerstrasse 2**
**D-81541 München (DE)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to photographic recording materials and more particularly to photographic recording materials having enhanced latent image keeping stability.

A visible image is formed in silver halide photographic materials by exposure of the material to actinic radiation to form a record of the exposure. This record, which is invisible to the unaided eye, yields a visible image by photographic processing of the exposed material.

It is generally believed that the latent image comprises minute specks of metallic silver. These specks form in or on individual silver halide grains by interaction between silver ions and photoelectrons generated by absorption of actinic radiation by the silver halide grains.

Processing of most common silver halide photographic materials includes a development step in which the material is contacted with an aqueous alkaline solution of a developing agent. The developing agent, which is a reducing agent, selectively reduces to metallic silver those silver halide grains containing a latent image.

It is recognized in the photographic art that a latent image is not permanent and that, with passage of time, silver halide grains which would be developable immediately after exposure become nondevelopable. This phenomenon is termed latent image fading and manifests itself as a loss in image density in the developed image and a consequent loss in speed in the silver halide photographic material.

If silver halide materials were developed immediately following imagewise exposure, latent image fading would not be a problem. However, with many silver halide materials, delays between exposure and processing frequently occur. For example, with amateur film materials in which multiple images are formed on a single roll of film there is often a delay of months between the time the first image is exposed and the time the exposed material is processed. With such materials latent image fading can present a significant problem and compounds are added to photographic materials to prevent or reduce this undesirable effect. These compounds are referred to as latent image stabilizers and prevention or reduction of latent image fading is referred to as latent image stabilization.

Various attempts have been made to solve this problem. One attempt is mentioned in U. S. Patent 3,954,478 where N-2-propenyl-benzothiazolium and N-2-propenyl-naphthothiazolium salts are described as being useful for this purpose.

Other proposed solutions to latent image instability are described in U. S. Patent 4,374,196 where acyclic compounds, obtained by basic hydrolysis of N-alkenylthiazolium salts, are shown to reduce latent image instability. In U.S. Patent 4,423,140 certain aromatic mercaptide compounds are described as being useful in reducing latent image instability. U.S. Patent 4,780,400 discloses 2-unsubstituted N-alkenylthiazolium salts as latent image stabilizers. Benzothiazolium salts containing ester groups in the N-substituent are also disclosed in U.S. Patent 4,948,721. Other latent image stabilizing compounds have also been described in U.S. Patent 4,378,426 and 4,451,557.

Notwithstanding these earlier attempts to reduce or to eliminate latent image instability, the need still exists in the photographic art for more effective stabilizers for latent images in exposed silver halide recording materials.

The present invention satisfies this need by providing a photographic recording material comprising a support and a photographic silver halide emulsion which has associated therewith a benzothiazolium or benzoselenazolium salt having the structural formula:

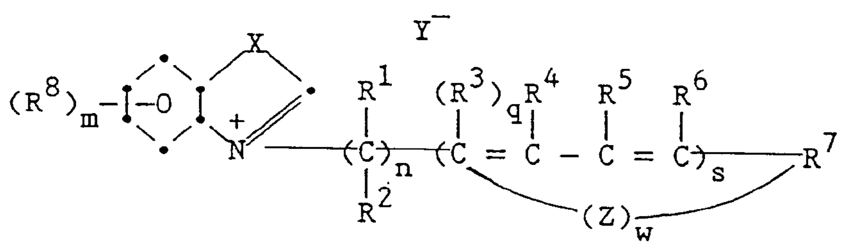

wherein:

X is sulfur or selenium;

Y is a counterbalancing ion;

m is 0 to 4;

n is 1 to 4;

w is 0 or 1, preferably 0;

s is 1 to 4, preferably 1 or 2;

q is 0 or 1, preferably 1;

$R^1$ and $R^2$ individually are hydrogen, unsubstituted or substituted alkyl containing 1 to 5 carbon atoms, such as methyl, ethyl, propyl, n-butyl, t-butyl or pentyl or aryl, such as phenyl; preferably hydrogen or methyl;

$R^3$, $R^4$, $R^5$ and $R^6$ individually are hydrogen or alkyl containing 1 to 5 carbon atoms, such as methyl, ethyl, propyl n-butyl or t-butyl; preferably hydrogen;

$R^7$ is unsubstituted or substituted alkyl or aryl; such as methyl, ethyl, propyl, n-butyl, t-butyl or phenyl; or $R^7$ with Z represents the atoms necessary to complete a cycloalkyl group when q is 0, such as $-CH_2-CH_2-$; $R^7$ is preferably methyl or ethyl;

$R^8$ is hydrogen; halogen, preferably chlorine or bromine; alkyl containing 1 to 5 carbon atoms, such as methyl, ethyl, propyl, n-butyl or t-butyl; or alkoxy containing 1 to 5 carbon atoms, such as methoxy, ethoxy, propoxy or butoxy; and

Z represents with $R^7$ the atoms necessary to complete a cycloalkyl group when q is 0.

The alkyl or alkoxy groups, as described, can be straight or branched chain. When $R^8$ is halogen it is preferably chloro.

The counterbalancing ion Y can be any anion compatible with the photographic material in which it is coated. Useful ions include inorganic anions such as halides, halophosphates, trifluoromethanesulfonates and the like.

While m can be an integer as high as 4, the preferred value is 1 or 2.

Specific compounds falling within this invention which can provide improved latent image stability in an exposed photographic silver halide emulsion include the following:

1.

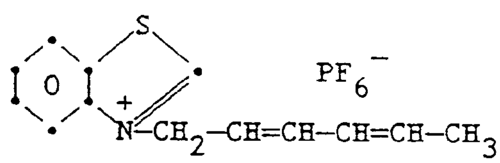

2.

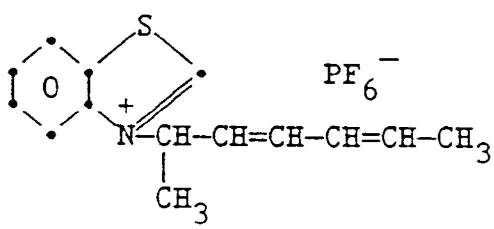

3.

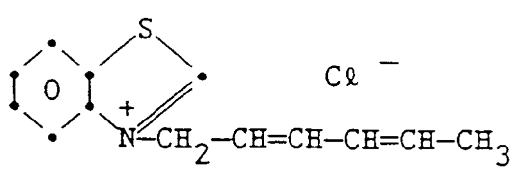

4.

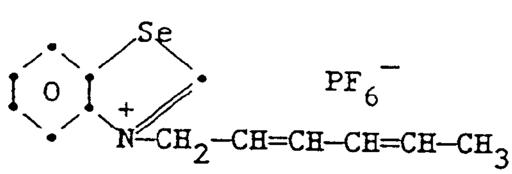

5.

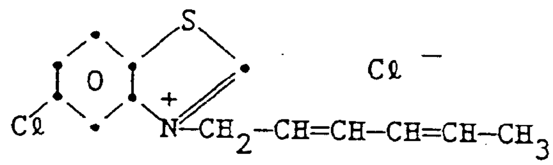

6.

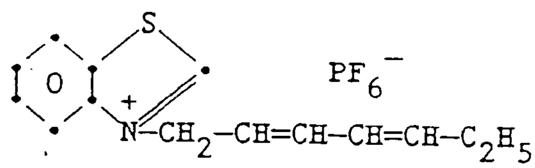

7.

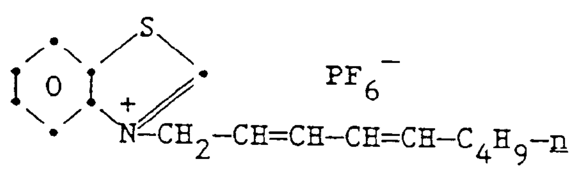

8.

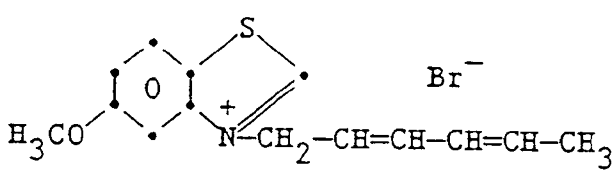

9.

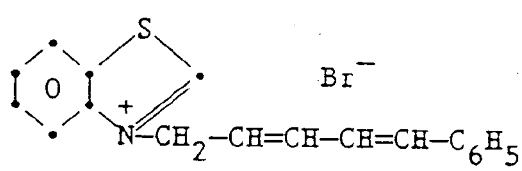

10.

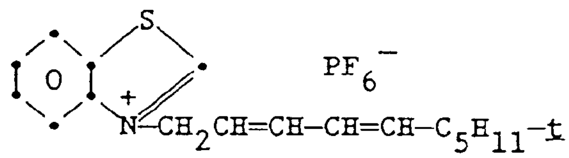

11.

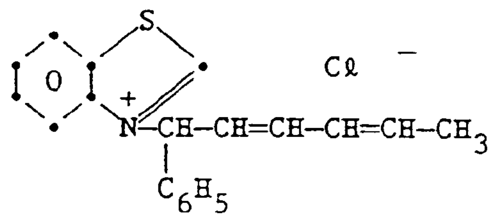

12.

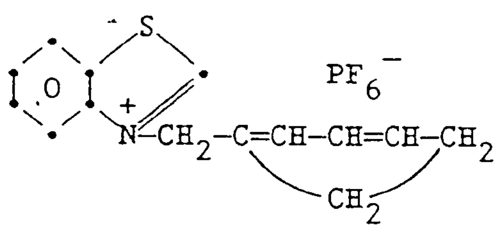

13.

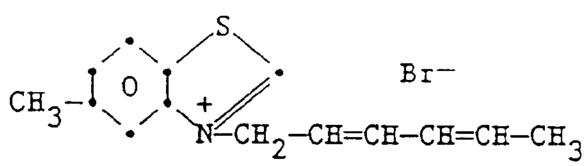

14.

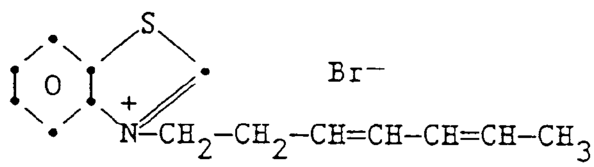

15. 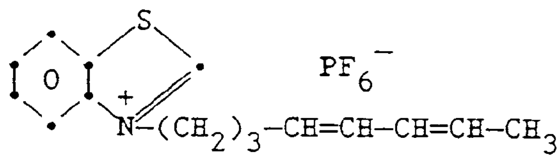

The dialkenyl benzothiazolium and benzoselenazolium compounds as described can be prepared by methods known in the organic compound synthesis art. A typical method of preparation is as follows: (Temperature herein are °C.)

General Method of Synthesis:

The benzothiazolium and benzoselenazolium compounds as described can be prepared by the route shown below. Commercially available or synthesized alcohols (I)

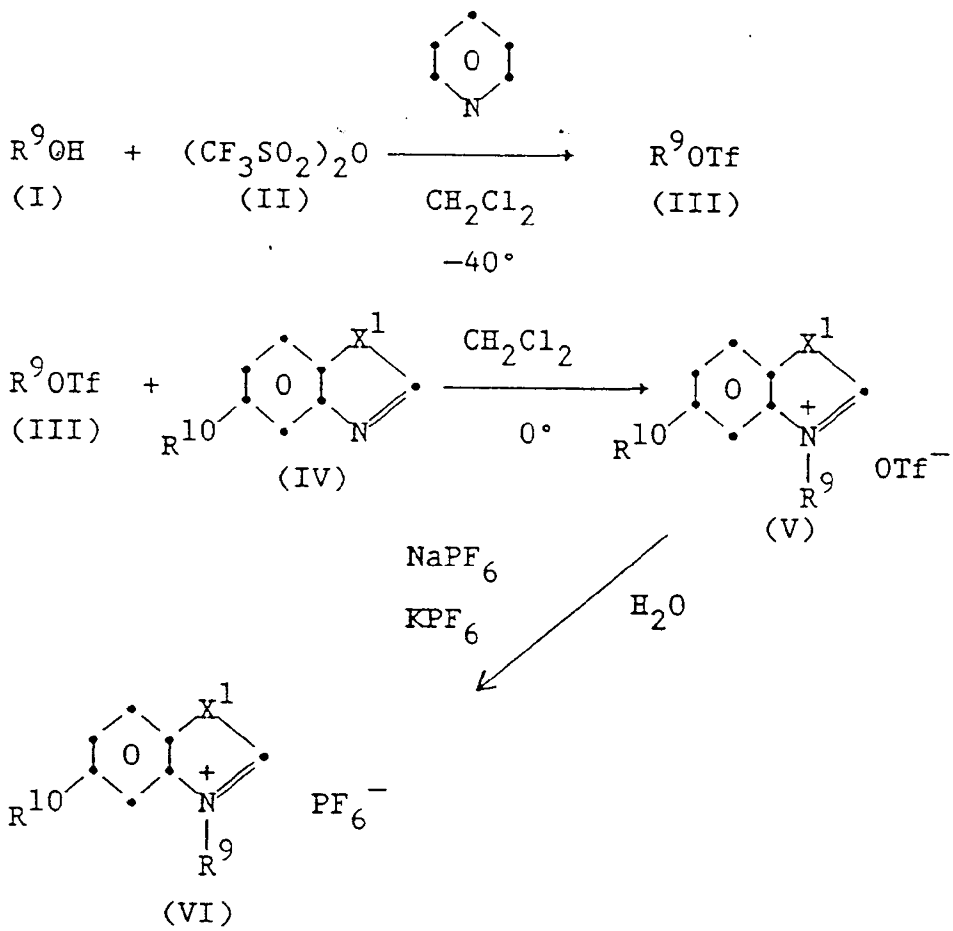


$R^9$ = dialkenyl group
$X^1$ = S, Se
Tf = $CF_3SO_2$
$R^{10}$ = H, halogen, alkyl, alkoxy group

were reacted with trifluoromethanesulfonic (triflic) anhydride (II) in the presence of pyridine at low temperature to form reactive triflates (III) in situ. The resulting reaction mixtures were filtered to remove pyridinium triflate and the solutions of (III) were immediately added to a solution of a benzothiazole or benzoselenazole (IV) at low temperature. The triflate salts (V) were formed after stirring for several hours. The solvent was removed and the remaining residue was dissolved in water. Addition of excess sodium or potassium hexafluorophosphate to the aqueous solution led to precipitation of the hexafluorophosphate salts (VI).

<u>Synthesis Example A</u> (Inventive Compound 1):

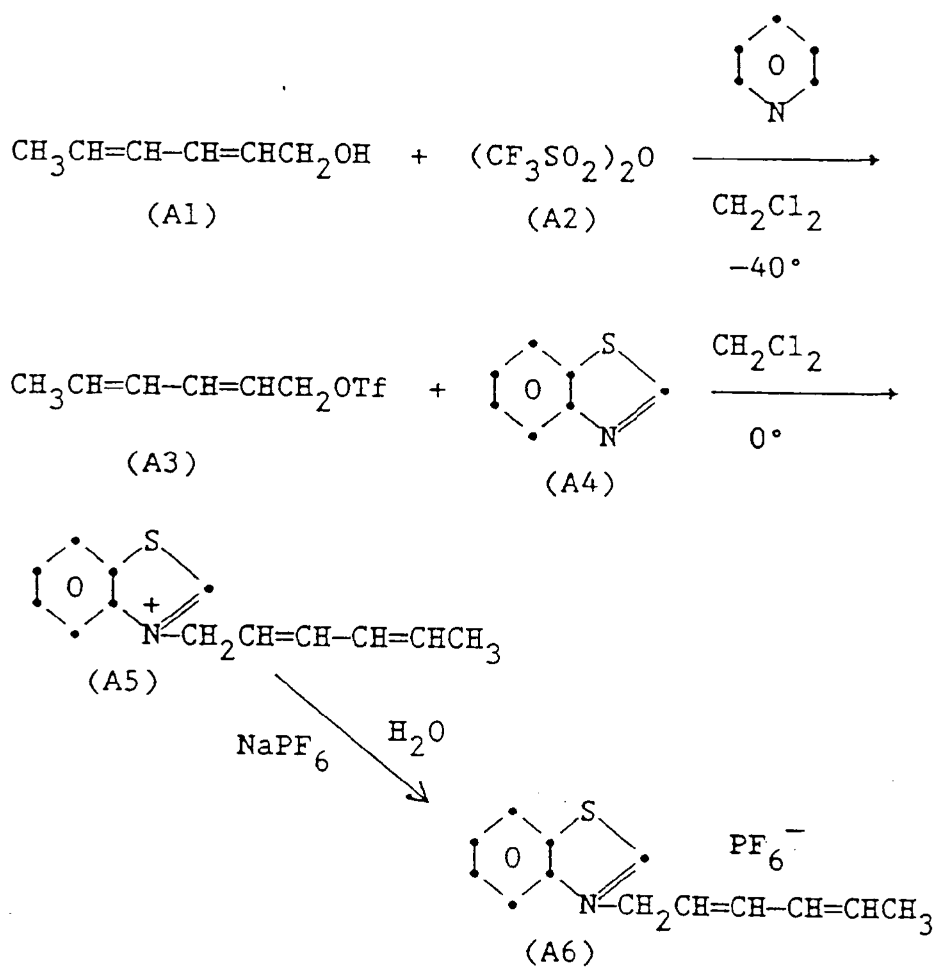


Pyridine (0.79g, 0.01 mole) and dichloromethane (20 mL) were mixed in a flask equipped with a magnetic stirrer, dropping funnel, nitrogen inlet, and reflux condenser. The flask was chilled in a dry ice - acetonitrile bath at -40° and a nitrogen atmosphere was maintained inside. A solution of triflic anhydride (A2) (2.82g, 0.01 mole) was added dropwise; a white precipitate formed. The resulting thick mixture was stirred for 10 min. at -40°. A solution of 2,4-hexadien-1-ol (A1) (0.98g, 0.01 mole) in dichloromethane (20 mL) was added dropwise over 40 min. After the addition was complete the mixture was stirred for 10 more min. at -40°. This mixture containing (A3) was filtered while still cold through a fritted glass funnel directly into another reaction flask containing benzothiazole (A4) (1.10g, 0.008 mole) and dichloromethane (40 mL). The flask was chilled in an ice bath and a nitrogen atmosphere was maintained inside. The reaction mixture was stirred for 2 hours at ice bath temperature and then for 16 hours at room temperature. The solvent was removed on a rotary evaporator. This gave (A5) as a tan oil. This material was stirred with water (25 mL). The resulting aqueous mixture was shaken with ether to remove any neutral material. The aqueous layer was transferred to a beaker and was stirred at room temperature with sodium hexafluorophosphate (1.0g) for 0.5 hour. The mixture was filtered and the collected solid was washed first with water, and then with ether. The product (A6) was dried overnight at room temperature in a vacuum oven. Yield 0.06g (2%) of cream colored powder, m.p. 103-106°. A nmr spectrum was consistent with structure (A6).

| | |
|---|---|
| Anal. Calcd. for $C_{13}H_{14}F_6NPS$: | C, 43.2; H, 3.9; N, 3.9. |
| Found: | C, 42.8; H, 3.9; N, 3.9. |

The benzothiazolium and benzoselenazolium salts described herein can be added to a silver halide emulsion at any point subsequent to precipitation of the silver halide grains. Preferably, the salts are added to an emulsion after chemical and spectral sensitization, but prior to coating. However, the salts can be present during these sensitization procedures.

The optimum amount of benzothiazolium or benzoselenazolium salt added to an emulsion will depend upon various factors such as the particular salt employed, the particular silver halide emulsion used or the nature of other components of the emulsion. Useful amounts are within the range of from about 0.002 to about 10 millimoles of salt per mole of silver. Preferably, the salt is incorporated in the emulsion in an amount of from about 0.02 to about 0.5 millimole per mole of silver.

The silver halide emulsions can be any of the silver halide emulsions known in the photographic art which are desirably protected against latent image instability or fading. The silver halide emulsions can be comprised of silver bromide, silver chloride, silver chlorobromide, silver chloroiodide, silver bromoiodide, silver chlorobromoiodide or mixtures thereof. The emulsions can include coarse, medium or fine grains and can be monodisperse or polydisperse.

The silver halide emulsions are preferably surface latent image-forming emulsions. They can be chemically sensitized as illustrated by T. H. James, The Theory of the Photographic Process, 4th Ed., MacMillan, 1977, pp. 67-76, or with sulfur, selenium, tellurium, gold, platinum, palladium, iridium, osmium, rhenium or phosphorus sensitizers, or combinations of these sensitizers, as illustrated by Research Disclosure, Vol. 134, June 1975, Item 13452, or in U. S. Patent Nos. 1,623,499; 1,673,522; 2,399,083; 2,642,361; 3,297,447; 3,297,446; 1,315,755; 3,772,031; 3,761,267; 3,857,711; 3,565,633; 3,901,714 and 3,904,415 and U. K. Patent No. 1,396,696.

Chemical sensitization can optionally be conducted in the presence of thiocyanate derivatives, as described in U. S. Patent Nos. 2,222,264 and 2,642,361; thioether compounds, as disclosed in U. S. Patent Nos. 2,521,926; 3,021,215 and 4,054,457; or azaindenes, azapyridazines and azapyrimidines, as described in U. S. Patent Nos. 3,411,914; 3,554,757; 3,565,631 and 3,901,714. Additionally or alternatively, the emulsions can be reduction sensitized e.g. , with hydrogen, as illustrated by U. S. Patent Nos. 3,891,446 and 3,984,249 by low pAg (e.g., less than 5), high pH (e.g., greater than 8) treatment or through the use of reducing agents, such as stannous chloride, thiourea diazide, polyamines and amineboranes, as illustrated by Research Disclosure, Vol. 136, August 1975, Item 13654, or U. S. Patent Nos. 2,518,696; 2,739,060; 2,743,182; 2,743,183; 2,983,609; 3,026,203 and 3,361,564 (Research Disclosure is published by Kenneth Mason Publications, Ltd., Dudley Annex, 12a North Street, Enisworth, Hampshire, PO107DQ England.)

The silver halide emulsions can be spectrally sensitized with dyes known in the photographic art, including the polymethine dye class, which includes the cyanines, merocyanines, complex cyanines and merocyanines (i.e., tri-, tetra-, and poly-nuclear cyanines and merocyanines), oxonols, hemioxonols, styryls, merostyryls and streptocyanines. Particularly useful dyes are benzoxazole, benzimidazole and benzothiazole carbocyanine dyes.

The photographic silver halide emulsions can contain various colloids alone or in combination as vehicles. Suitable hydrophilic materials as well as hardeners therefore, are described in Research Disclosure, December 1989, Item 308119, Sections IX and X.

The photographic silver halide emulsions and elements employing the stabilizing agents of this invention can contain other addenda conventional in the photographic art. Useful addenda are described, for example, in Research Disclosure, December 1989, Item 308119 and include antifoggants, couplers (such as dye forming couplers, masking couplers, DIR and DIAR couplers) DIR compounds, anti-stain agents, image dye stabilizers, absorbing materials such as filter dyes and UV absorbers, light scattering materials, coating aids, plasticizers and lubricants.

The photographic recording materials described herein can be black-and-white or monochrome materials or they can be multilayer and/or multicolor elements comprising a support bearing one or more layers of a silver halide emulsion. These materials can be designed for processing in conventional developer solutions. Multicolor elements can contain dye image forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art. In an alternative format, the emulsion or emulsions can be disposed as one or more segmented layers.

A preferred color photographic recording material according to this invention comprises a support bearing at least one blue-sensitive silver halide emulsion layer having associated therewith a yellow dye-forming coupler, at least one green-sensitive silver halide emulsion layer having associated therewith a magenta dye-forming coupler and at least one red-sensitive silver halide emulsion layer having associated therewith a cyan dye-forming coupler, at least one of the silver halide emulsion layers containing a latent image stabilizing compound of this invention. In accordance with a particularly preferred aspect of the present invention, the stabilizing compound is contained in a yellow dye-forming blue-sensitive silver halide emulsion.

The photographic recording materials of the present invention can contain additional layers conventional in photographic elements, such as overcoat layers, spacer layers, filter layers, antihalation layers, scavenger

layers and the like. The support can be any suitable support used with photographic elements. Typical supports include polymeric films, paper (including polymer-coated paper), glass and the like. Details regarding supports and other layers of the photographic elements of this invention are contained in Research Disclosure, December 1989, Item 308119, referred to above.

As used herein, the term "associated therewith" signifies that the stabilizing compound is in a silver halide emulsion layer or in an adjacent layer so that the materials contained therein are accessible to one another.

The following examples further illustrate this invention.

Example 1 - Color Photographic Evaluation

Benzothiazolium salts as latent image keeping agents were evaluated in a color negative test. The emulsion I used was a 1.6 micrometer equivalent circular diameter, .12 micrometer thick tabular silver bromoiodide emulsion containing 6 mol % iodide. The emulsion was sulfur and gold sensitized with a yellow optical sensitizing dye. This emulsion was coated in a two layer monochrome with a protective overcoat over the image layer. The image layer contained the emulsion, a yellow dye forming coupler, and the latent image keeping agents coated both at .02 and .10 mmole/mole Ag. The melt pH was 5.9 and the melt pAg was 7.9. The latent image keeping (LIK) coatings were exposed for 1/100 second and were incubated at 25°C, 50 % RH for four weeks.

A similar color negative test was carried out with a similar silver bromoiodide emulsion (Emulsion II) containing 3 mol % iodide.

The check coatings were stored at -18°C and then were exposed. Both coatings were processed in a C-41 process with a commercial bleach (C-41 is a tradename and available from Eastman Kodak Company, U.S.A.). The processed coatings were read with status M densitometry. Results are reported in Table 1.

Table 1

| | | mmole / Ag mole | Fresh Speed | 4 wk RSK Speed | 4 wk LIK Speed | Delta Speed |
|---|---|---|---|---|---|---|
| Emulsion I | Control | - | 240.7 | 242.9 | 225.8 | -14.9 |
| | Compound A* | .10 | 244.2 | 245.0 | 232.2 | -11.0 |
| | Compound B | .10 | 246.2 | 245.7 | 240.0 | - 6.2 |
| | Compound C* | .02 | 245.2 | 244.5 | 239.7 | - 5.5 |
| Emulsion II | Control | - | 190.8 | 193.7 | 174.6 | -16.2 |
| | Compound A* | .10 | 193.8 | 195.2 | 180.0 | -13.8 |
| | Compound B | .10 | 205.8 | 205.8 | 203.1 | - 2.7 |
| | Compound C* | .02 | 218.1 | 215.8 | 211.3 | - 6.8 |

Delta Speed: 4 wk LIK Speed - Fresh Speed

* = comparison
RSK = raw stock keeping
LIK = latent image keeping

The compounds A, B and C are as follows:

Compound A

N-allyl benzothiazolium

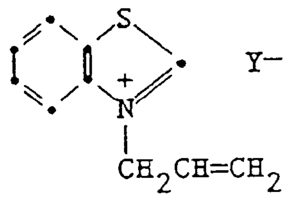

Compound B (Inventive Compound 1)

2H-N-(2,4-hexadiene-1-yl)-benzothiazolium

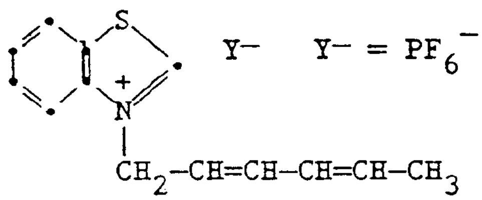

Compound C

2-propargylaminobenzoxazole

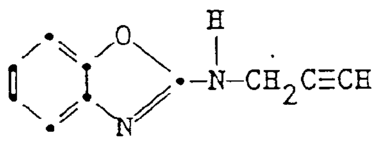

Data in Table 1 show that there is reduced latent image loss in the presence of a benzothiazolium salt containing a dialkenyl group as compared with a control having no agent.

## Claims

1. A photographic recording material comprising a support and a silver halide emulsion which has associated therewith a benzothiazolium or benzoselenazolium salt having the structural formula:

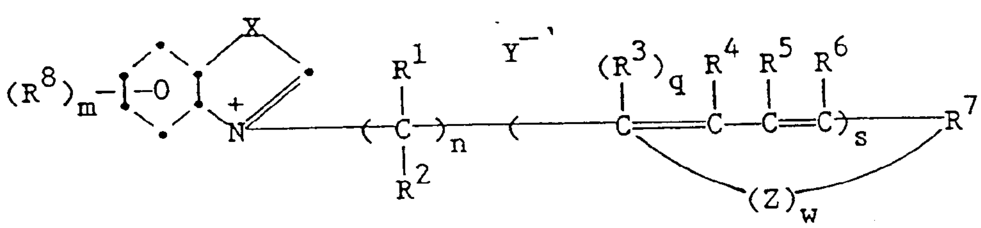

wherein:

X is sulfur or selenium;

Y is a counterbalancing ion;

m is 0 to 4;

n is 1 to 4;

w is 0 or 1;

s is 1 to 4;

q is 0 to 1;

$R^1$ and $R^2$ individually are hydrogen, unsubstituted or substituted alkyl containing 1 to 5 carbon atoms, or aryl;

$R^3$, $R^4$, $R^5$ and $R^6$ individually are hydrogen or alkyl containing 1 to 5 carbon atoms;

$R^7$ is unsubstituted or substituted alkyl or aryl; or $R^7$ with Z represents the atoms necessary to complete a cycloalkyl group when q is 0;

$R^8$ is hydrogen, halogen, alkyl containing 1 to 5 carbon atoms, or alkoxy containing 1 to 5 carbon atoms;

Z represents with $R^7$ the atoms necessary to complete a cycloalkyl group when q is 0.

2. The recording material as claimed in claim 1 wherein $R^8$ is chlorine.

3. The recording material as claimed in claim 1 wherein $R^8$ is alkyl or alkoxy having from 1 to 5 carbon atoms.

4. The recording material as claimed in claim 1 wherein x is sulfur; $R^8$ is hydrogen; $R^1$ and $R^2$ are hydrogen; q is 1; $R^3$, $R^4$, $R^5$ and $R^6$ are hydrogen.

5. The recording material as claimed in any of claims 1 - 4 wherein the salt is present in an amount of from 0.02 to about 0.5 millimole thereof per mole of silver.

6. The recording material as claimed in any of claims 1 - 5 which is a color photographic recording material.

7. The recording material as claimed in any of claims 1 and 5 -6 wherein the salt has the structural formula:

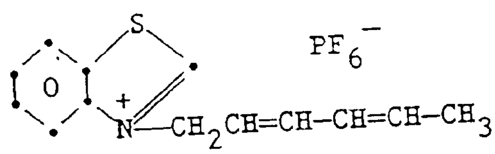

## Patentansprüche

1. Photographisches Aufzeichnungsmaterial mit einem Träger und einer Silberhalogenidemulsion, der ein Benzothiazolium- oder Benzoselenazoliumsalz der folgenden Strukturformel zugeordnet ist:

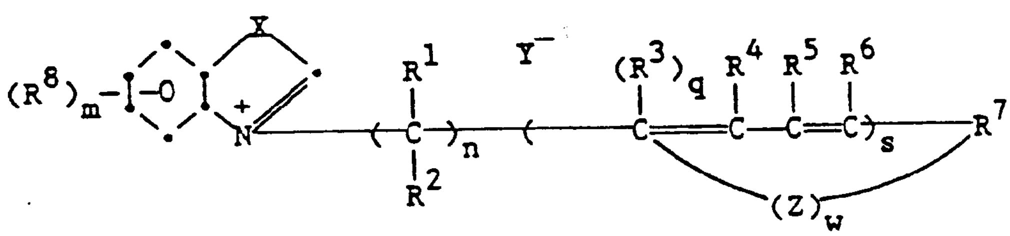

worin bedeuten:
X gleich Schwefel oder Selen;
Y ein Ladungen ausgleichendes Gegenion;
m gleich 0 bis 4;
n gleich 1 bis 4;
w gleich 0 oder 1;
s gleich 1 bis 4;
q gleich 0 bis 1;
$R^1$ und $R^2$ einzeln gleich Wasserstoff, unsubstituiertes oder substituiertes Alkyl mit 1 - 5 Kohlenstoffatomen oder Aryl;
$R^3$, $R^4$, $R^5$ und $R^6$ einzeln Wasserstoff oder Alkyl mit 1 bis 5 Kohlenstoffatomen;
$R^7$ unsubstituiertes oder substituiertes Alkyl oder Aryl; oder $R^7$ mit Z steht für die Atome, die zur Vervollständigung einer Cycloalkylgruppe erforderlich sind, wenn q gleich 0 ist;
$R^8$ gleich Wasserstoff, Halogen, Alkyl mit 1 bis 5 Kohlenstoffatomen oder Alkoxy mit 1 bis 5 Kohlenstoffatomen;
Z steht mit $R^7$ für die Atome, die zur Vervollständigung einer Cycloalkylgruppe erforderlich sind, wenn q gleich 0 ist.

2. Aufzeichnungsmaterial nach Anspruch 1, in dem $R^8$ für Chlor steht.

3. Aufzeichnungsmaterial nach Anspruch 1, in dem $R^8$ für Alkyl oder Alkoxy mit 1 bis 5 Kohlenstoffatomen

steht.

4. Aufzeichnungsmaterial nach Anspruch 1, worin X für Schwefel steht, $R^8$ gleich Wasserstoff ist; $R^1$ und $R^2$ Wasserstoffatome darstellen, q gleich 1 ist; $R^3$, $R^4$, $R^5$ und $R^6$ für Wasserstoffatome stehen.

5. Aufzeichnungsmaterial nach einem der Ansprüche 1 bis 4, in dem das Salz in einer Menge von 0,02 bis etwa 0,5 Millimolen pro Mol Silber vorliegt.

6. Aufzeichnungsmaterial nach einem der Ansprüche 1 bis 5, das ein farbphotographisches Aufzeichnungsmaterial ist.

7. Aufzeichnungsmaterial nach einem der Ansprüche 1 sowie 5 bis 6, in dem das Salz der folgenden Strukturformel entspricht:

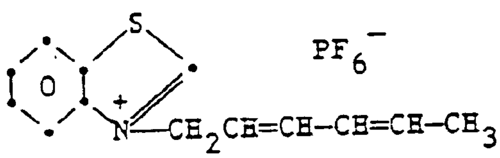

**Revendications**

1. Produit d'enregistrement photographique comprenant un support et une émulsion aux halogénures d'argent à laquelle est associé un sel de benzothiazolium ou de benzosélénazolium de formule :

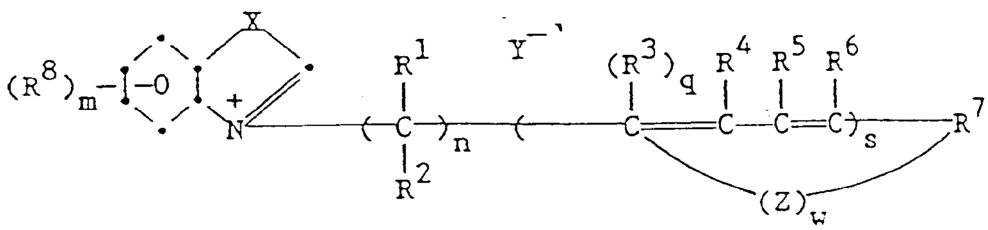

où

X est du soufre ou du sélénium;
Y est un ion pour équilibrer la charge ionique ;
m est de 0 à 4 ;
n est de 1 à 4 ;
w est de 0 ou 1 ;
s est de 1 à 4 ;
q est 0 ou 1 ;
$R^1$ et $R^2$ chacun séparément sont l'hydrogène, un groupe alkyle substitué ou non de 1 à 5 atomes de carbone, ou aryle ;
$R^3$, $R^4$, $R^5$ et $R^6$ chacun séparément sont l'hydrogène, un groupe alkyle de 1 à 5 atomes de carbone ;
$R^7$ est un alkyle ou aryle substitué ou non ; ou $R^7$ avec Z représente les atomes nécessaires pour compléter un groupe cycloalkyle quand q est 0 ;
$R^8$ est l'hydrogène, halogène, alkyle contenant de 1 à 5 atomes de carbone ou alkoxy contenant de 1 à 5 atomes de carbone;
Z représente avec $R^7$ les atomes nécessaires pour compléter un groupe cycloalkyle quand q est 0.

2. Produit d'enregistrement photographique selon la revendication 1 dans lequel $R^8$ est le chlore.

3. Produit d'enregistrement photographique selon la revendication 1 dans leques $R^8$ est un alkyle ou un alkoxy ayant de 1 à 5 atomes de carbone.

4. Produit d'enregistrement photographique selon la revendication 1 dans lequel X est le soufre ; $R^8$ est l'hydrogène ; $R^1$ et $R^2$ sont l'hydrogène ; q est 1 ; $R^3$, $R^4$, $R^5$, et $R^6$ sont l'hydrogène.

5. Produit d'enregistrement photographique selon les revendications 1 à 4 dans lequel le sel est présent à raison de 0,02 à 0,5 millimole par mole d'argent.

6. Produit d'enregistrement photographique selon les revendications 1 à 5 qui est un produit en couleurs.

7. Produit d'enregistrement photographique selon l'une quelconque des revendications 1, 5 ou 6 dans lequel le sel a la formule :